# EUROPEAN PATENT APPLICATION

(11) **EP 4 682 590 A1**
(43) Date of publication of application: **21.01.2026**
(21) Application number: 25189466.3
(22) Date of filing: 15.07.2025
(51) Int. Cl.: G01T 1/164, G01T 1/29, G01T 7/00, A61B 6/58, G01T 1/161, A61B 6/03, A61B 6/42, A61N 5/10

(54) **PET DETECTOR CALIBRATION METHOD AND DEVICE FOR CALIBRATION**

(30) Priority: 15.07.2024 ES 202430602
(71) Applicant: Bruker Española, S.A., 28521 Rivas-Vaciamadrid Madrid (ES); Consejo Superior De Investigaciones Científicas - CSIC, 28006 Madrid (ES); Universitat Politecnica de Valencia, 46022, Valencia (ES)
(72) Inventor: MOLINER MARTINEZ, Laura, 12600 Castellón (ES); BENLLOCH BAVIERA, José María, 46003 Valencia (ES); SALVADOR CORRECHER, Carlos, 12592 Xilxes (Castellón) (ES); BELTRÁ PICÓ, Adrián, 03650 Pinosó (Alicante) (ES); BRUYNDONCKX, Peter, 1980 Eppegem (Belgium) (BE)
(74) Representative: Cueto, Sénida

(57) **Abstract**

A calibration method for a PET apparatus comprising a set of paired opposing PET detectors, comprising:
1) - disposing a movable assembly comprising a radioactive source and an external gamma ray detector, the radioactive source being close to, and aligned with, a first detector of the PET apparatus,
2) - remote positioning of the gamma ray detector close to a second PET detector, the gamma ray detector being on an axis with the radioactive source perpendicular to calibration axis,
3) - exposing the detector to calibrate and the external detector to the radioactive source,
4) - shifting the movable assembly along the axis of calibration, in a number of discrete steps, for each step:
- obtaining event data
- recognizing and storing coincidence t events
- linking event data to a spatial region in the scintillation crystal
9 - moving the mechanical movable assembly in the PET apparatus such that the radioactive source comes close to another detector.

## Description

### FIELD OF THE INVENTION

The present invention relates to the field of detectors for emission tomography. More specifically, it relates to a method and device for performing a self-calibration of a monolithic or semi-monolithic crystal-based detector for emission tomography, more particularly for PET.

### BACKGROUND OF THE INVENTION

PET imaging is a non-invasive medical technique that provides detailed information about the biological processes inside the body. This image modality relies on the detection of positron-emitting radionuclides, which are injected into the patient's body. The injected radionuclides undergo positron decay, resulting in positron-electron annihilation process and therefore, emitting two gamma photons in opposite directions at 511 keV energy each. An event in PET (coincidence) consists in the simultaneous detection of these two annihilation photons and, after the reconstruction process, a functional image that represents the radiotracer distribution inside the body is obtained. The detectors are usually constructed using a scintillator crystal that will turn the gamma ray energy into visible light, connected to an array of photosensors (as PMTs or SiPM) that will convert the light into electrical signals. These electrical signals are digitalized to obtain information of the light distribution that is then processed to finally provide us the 3D impact position and the deposited energy of the incident gamma photons in the scintillator crystals.

There also exist one step detectors that use semiconductor technology to directly convert the incident gamma photons' energy into electrical signals, providing position and energy information of these interactions.

To generate high quality images, the accuracy of the photon impact position and its energy determination is crucial. The error in the location of these events will result into mispositioning of the Line-Of-Response (LOR), the theoretical line that joins the two photons, result of the annihilation, impacts points in the opposite detectors that provides the basic information used for the reconstruction algorithms to generate the image. This mispositioning will result in a blurrier and less accurate image. On the other hand, gamma photons that have suffered scattering cannot be estimated correctly if the energy deposited is not measured with the required precision, ultimately affecting the quantification of the final reconstructed image.

Many systems use pixelated detectors, where the problem to determine in which pixel the gamma ray was stopped is simplified. Even in these cases, some calibration of the individual detectors is needed, as the crystals are usually not coupled to photo-sensors one-by-one, so the light generated in the crystals is distributed among several photo-sensors. Additionally, the data have to be digitalized, introducing noise and then requiring some positioning calibration to ensure that the real crystal is identified after the processing. These pixelated detectors are very efficient in the position localization but does not allow to determine the depth-of-interaction in the crystal. More advanced systems as the phoswich technology (two layers of pixels), or dual-readout systems (photo-detectors in both sides of the crystals) allow at least a partial depth-of-interaction information but increase the complexity and the cost. In these cases, also a specific calibration is needed to recover the full 3D impact position.

The positioning determination became more complex when using monolithic or semi-monolithic detectors, where the distribution of light generated by the gamma ray detection travels through the crystal and deposits light in several photosensors. In this case this light distribution needs to be characterized to recover the 3D positioning. Many algorithms have been published to deal with the light distribution data, from variations of the center of gravity location (Center of Gravity (CoG) or Raise-To-the-Power (RTP) methods) to machine learning methods as tree-boosting, Maximum Likelihood Expectation Maximization (MLEM) or neural networks.

In all cases, regardless of the method to be used, some calibration procedure needs to be done, usually taking some reference data and using it to generate look-up-tables, probability models or neural network models. The procedure to acquire calibration data is crucial to obtain good quality reference data to provide a precise data model to be used in the positioning algorithm and energy estimation.

There are several methods in the state-of-the-art used to obtain calibration data. The most common is the use of collimated sources. This procedure exposes the detector to a collimated source, where the incoming gamma ray positions and direction are known. The electrical signals generated can be then characterized and mapped (depending on the positioning algorithm) to the real impact position. The source is then moved to several reference positions (can be in all three dimensions of the sensor) and the reference data used to generate the complete model of the detector. This procedure is time consuming and requires mechanical means with high precision (for example, a robotic arm) to automate the process.

This procedure can be simplified using 2D-mask where the collimated source provides parallel beams to different positions of the detector, reducing the time of calibration, but increasing the collimator weight and then the positioning complexity of the calibration assembly.

A variation of the collimated source is the use of collimated beams, where each beam can be displaced through one plane of the detector (e.g. one fan in the xz-plane, another in the yz-plane and, possibly, a third in the xy-plane) providing information of a complete detector line, not just one point as the collimated source. This can reduce the procedure time but increases the complexity of the algorithms to generate the full detector model. These methods present several drawbacks:
- The detectors need to be calibrated individually before they are mounted in the final system as there is no sufficient space (specially in small animal systems) when the full detector arrangement is assembled. It is specially complicated to calibrate the z-axis (depth-of-interaction) when the system is fully mounted.
- High activity sources are needed, or liquid sources need to be re-filled several times in order to have enough information for the calibration process, due to the use of the collimators.
- The collimators generate scatter that is an unavoidable source of noise that is difficult to identify and correct, increasing the energy resolution and positioning errors.
- The weight of the collimators increases the requirements of the mechanical means for moving the calibration assembly to different locations, making impossible to use low-cost robotic arms that can hold only 1-2 kg weight.

On the other hand, there also are procedures that can make use of non-collimated sources (US10393895) where the detector is exposed to radiation from a non-collimated source, such as a point source, and gathering numerous samples without specific targeting. These samples can then undergo additional processing utilizing a self-organizing method like Kohonen's self-organizing map (SOM) [Kohonen, T. Self-organized formation of topologically correct feature maps. Biol. Cybern. 43, 59-69 (1982). https://doi.org/10.1007/BF00337288].

This approach has the advantage of using a much simpler mechanics (just the point source inside the system, already assembled), but requires a much more complex procedure, Monte Carlo simulations to predict the scintillation events are required, a very large calibration set is needed (maybe just one source is not sufficient) and the positioning error of the source is a major source of errors in the calibration. This method has not been used to calibrate the time-walk in the detector, which will add new complexity to the calculations.

The present invention provides a calibration method that avoids the use of heavy collimators and complex processing algorithms. The invention also provides a calibration device that includes an additional detector (with respect to the detectors to be calibrated), very narrow in the calibration direction, that will select only the proper events for calibration by means of electronic collimation.

The provided information is similar to the fan-beam calibration sources without the unwanted scatter effects and with the advantage of using a very light-weight calibration system that could easily be used in the installation sites of the scanners (even for small animal systems). This invention allows in-system calibration, that is, when the system is already assembled.

### Description of the invention

The present invention refers to a calibration method for a **PET apparatus** comprising a set of paired opposing PET detectors, the method comprising:
1) - disposing **a mechanical movable assembly** comprising a **radioactive source** that emits positrons and **an external gamma ray detector,** where the radioactive source is located close to, and aligned with, **a first detector of the PET apparatus** to be calibrated,
2) - remote positioning of the external gamma ray detector close to a **second detector of the PET apparatus** which is opposite to the first detector, the external gamma ray detector being on an axis with the radioactive source that is perpendicular to an axis of calibration,
3) - exposing the detector to be calibrated and the external gamma-ray detector to radiation of the radioactive source,
4) - shifting the mechanical movable assembly by means of **a first mechanical arrangement,** allowing the movement of the radioactive source and the external gamma ray detector as a unit, along the axis of calibration, in a number of discrete steps, for each step:
   5) - obtaining coincidence event data of the radioactive source, from the detector to be calibrated and the external gamma ray detector,
   6) - recognizing and storing coincidence t events between the detector to be calibrated and the external gamma ray detector,
   7) - linking event data to a spatial region in the scintillation crystal of the detector to be calibrated, and
8 - moving the mechanical movable assembly within the PET apparatus as a unit such that the radioactive source comes close to another detector to be calibrated, repeating steps 4-7 for each detector to be calibrated in the PET apparatus.

With regard to step 5) of the method, for the PET coincidence events to occur, i.e. two gamma rays being detected in a very short time period, the detector to be calibrated and the external gamma ray detector have to be facing each other, and only in this way data can be obtained from the two opposed detectors. Obtaining event data from detectors is the basic mechanism of the system, and an essential method step.

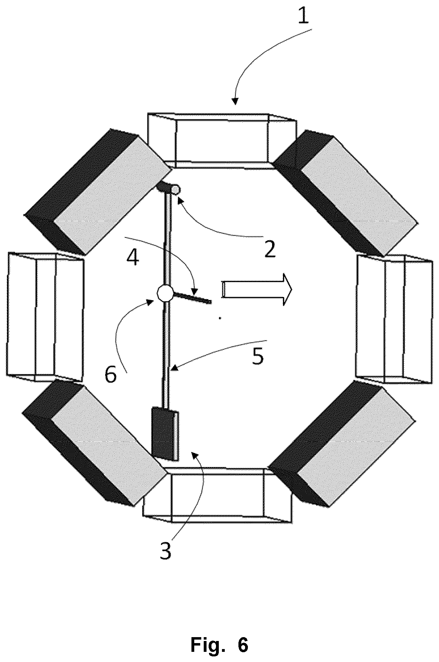

With regard to step 6 of the method, in Figure 6 of the present specification, it can be seen that the mechanical movable assembly is moved within the space surrounding the PET detectors, which form a kind of ring. It can be said that the mechanical movable assembly moves within this ring, and the method could not be carried out otherwise.

The method of the invention can be called an "in-system calibration method" since it is carried out with the apparatus completely assembled, that is, as it has to be ready for use.

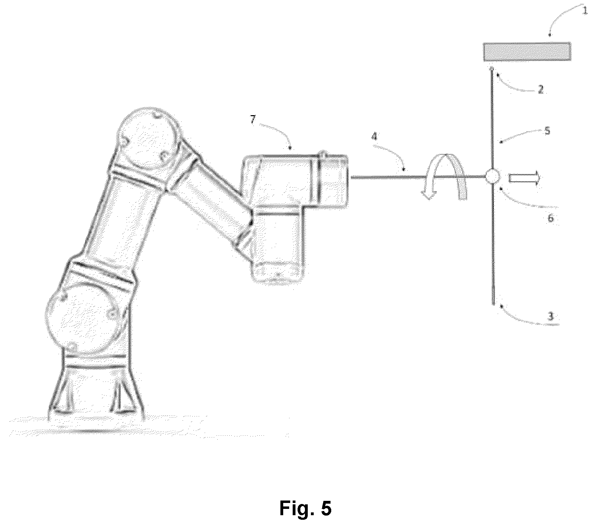

With regard to step 8) of the method, it can referto the rotation of the mechanical movable assembly. In Figure 5, an arrow shows the rotation. In Figure 6 it can be seen a PET apparatus with a ring configuration where the system will rotate from one detector (detector 1, represented as transparent in the Figure) to the next one (represented as shadowed in the Figure) to accomplish step 8).

The method of the invention can further comprise:
9. In case of the need to calibrate another detector axis, rotating the radioactive source and the external gamma ray detector as a unit, aligning them with a second calibration direction, and repeating steps 4-7 to calibrate the complete set of detectors in their final geometry for their use.

The method of the invention can further comprise:
10. In case of the need of depth-of-interaction calibration, rotating the radioactive source and the external gamma ray detector as a unit, aligning them in a direction not-perpendicular to surface of the detector to be calibrated. Repeat steps 4-7 to calibrate all the detectors in the final geometry for their use.

Optional step 9 and 10 can be carried out after step 8 of the method, independently from each other, or can be carried out both sequentially.

The method of the present invention needs a radioactive source, since it is based on the exclusive detection of coincidences with the external gamma ray detector. This is the basis of electronic calibration, without shielding.

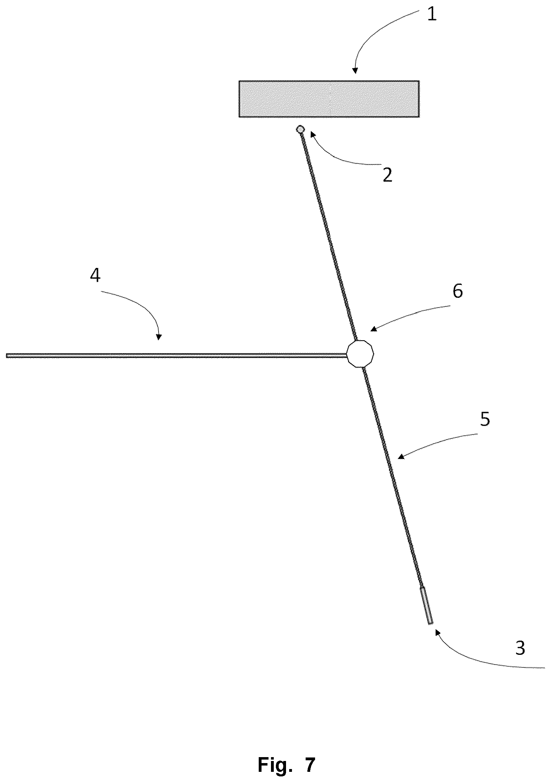

A mechanical arrangement allows the movement of the mechanical movable assembly lineally in the three-space axes and also its rotation in the axial axis and/or the other axis (to, for example, acquire data at 45 degrees for DOI calibration). This is, it can rotate along the axial axis or any other axis. Rotation along the axial axis is described as step 8, and rotation along any of the other axes allows for step 10 to take place (this rotation can be seen in Figure 7, wherein the mechanical movable assembly is rotated an axis that is not perpendicular to the axial axis.

According to preferred embodiments, the calibration is carried out for the three axes, as can be seen in the accompanying figures. For each calibration axis, the mechanical movable assembly rotates in the axial axis to calibrate all the PET detectors and moves in the calibration axis (axial or transaxial - shown in Figure 5 by the arrow above element 4).

According to additional embodiments, one or more additional calibration devices are used, such that each one has a mechanical assembly comprising a radioactive source and an external gamma ray detector in the appropriate orientation to calibrate a specific axis and the mechanical movable assembly only has to move axially and rotate transaxially.

The invention also refers to a calibration device for a PET apparatus to carry out the method previously described, for a group of gamma detectors arranged in the final geometry for their use, the device comprising:
- a radioactive positron source, with a thickness in the axis of the calibration smaller than the required calibration precision, an external gamma ray detector which forms a mechanical movable assembly with the radioactive source,
- a first mechanical arrangement with capacity to move the mechanical movable assembly, allowing the axial displacement and axial rotations of the mechanical movable assembly,
- a coincidence processor to detect and store coincidences between the detector to be calibrated and the external gamma ray detector and
- a data processor to take into account the acquired data at each calibration step and generates position calibration information for the calibrated detector,
- a rigid mechanical support that connects the mechanical movable assembly with the mechanical means for its displacement and rotation.

Step 5) of the method refers to the fact that data must be obtained, without specifying how they are obtained. The device comprises a coincidence processor as the generic means for obtaining data, since depending on the electronics, coincidence detection can be done in different ways. The alternative of using separated data from the detectors (singles) and a common clock for obtaining data is one alternative among others.

It is necessary in step 5) for the obtaining of data that the detector to be calibrated and the external gamma ray detector are facing each other in order the coincidence event to occur.

The radioactive source is a source that emits positrons, and is located close to the detector to be calibrated.

The source of positrons can be a point, a line source or a liquid source concentrated, that emits positron or gamma rays, located close to the detector to be calibrated.

The external gamma ray detector width (thickness in the calibration direction) will determine the precision of the calibration, usually it will be 1-2mm.

The external gamma ray detector forms a mechanical movable assembly with the radioactive source and it is perfectly aligned to the radioactive source and located on the opposite side of the system to be calibrated, as far as possible from the radioactive source.

The group of gamma detectors can comprise two-step detectors or one-step detector (semiconductors).

The detectors of the PET apparatus are arranged in the final geometry for their application. This geometry can be, for example:
▪ Ring geometry.
▪ Opposite pallets
▪ Open geometries

The first mechanical arrangement allows the movement of the radioactive source and the external gamma ray detector lineally in the three-space axis.

According to particular embodiments, the second mechanical arrangement also allows rotation of the mechanical movable assembly around the axial axis to locate it close to the detector to be calibrated in the final detector arrangement. It also can allow the rotation around the transaxial axis (to for example to acquire data at 45 degrees for DOI calibration).

Thanks to the mechanical arrangement allowing the movement of the radioactive source and the external gamma ray detector, each position acquired by the source will be a point for calibration, the calibration steps will be as small as the precision required for the calibration.

The data processor will use the acquired data at each calibration step and generates position calibration information for the calibrated detector based on one of the following known techniques:
▪ Center of gravity approached.
▪ Look-up tables.
▪ Neural Networks (or AI approaches).
▪ Statistical algorithms.
▪ Function fits (monomial, non-lineal functions, etc..).

A monolithic scintillation crystal means an undivided or continuous block of scintillator material read out by an array of photosensitive sensor devices. The scintillation photons generated in the monolithic scintillation crystal can travel completely free inside a scintillator block and the light is spread all over the array of photosensitive sensor devices.

Pixelated or segmented scintillation crystals mean comprise a plurality of small scintillation crystal tubes separated from each other with reflective material. The scintillation light generated in such a segmented or pixelated scintillation crystal is therefore transmitted and focused into a small area of the array of photosensitive sensor devices.

According to additional particular embodiments, the device comprises LYSO crystal and SiPM photosensors.

According to additional particular embodiments the device comprises an acquisition system that acquires all the singles information for both the electronic readout and the PET apparatus with a common reference clock.

According to additional particular embodiments the first mechanical arrangement is an articulated robotic arm.

According to additional particular embodiments the data processor is capable of using the acquired data to calculate the PET coincidence events and can determine the time skew of a detector after a scan of the detector surface.

According to additional particular embodiments the data processor is capable of using the acquired data to calculate the PET coincidence events and can determine the energy resolution of a detector after a scan of the detector surface.

According to additional particular embodiments the data processor is capable of using the acquired data to calculate the PET coincidence events and can determine the neural network parameters of a neural network model that provides the planar position of the impacts of a detector after a scan of the detector surface.

According to additional particular embodiments the data processor is capable of using the acquired data to calculate the PET coincidence events and can determine the neural network parameters of a neural network model that provides the DOI of the impacts of a detector after a scan of the detector surface, having the external gamma ray detector rotated in one of the transaxial axes.

According to additional particular embodiments the calibration device calculates alternative positioning models such as center-of-gravity, machine learning algorithms, look-up tables, statistical algorithms or specific functions.

The calibration device can be very light-weight as the mechanical supports need to be manufactured with low-attenuation materials, such as plastics and the radioactive source and external gamma ray detector will have a weight only of hundreds of grams. This provides a big flexibility to select an automated system (as a robotic arm or assembly of linear actuators) to provide high positioning accuracy of the device (an also with a low-cost mechanics).

If two-step detector is used, the scintillator crystal used for the external gamma ray detector can be the same scintillator crystal as the one used in the set of detectors to be calibrated or another one, that can, for example have higher stopping power or much better time resolution (as LaBr₃ or BaF₂) as it will be only needed to detect the time of the coincidence event with disregard of the actual position in the external gamma ray detector.

In the current generation of time-of-flight electronics the systems usually digitalize all singles information of all of the detectors and the coincidence processing is done with software or firmware. In this scheme is very easy to add a new detector in the workflow to be processed as one of the detectors embedded in the system.

### Brief description of figures

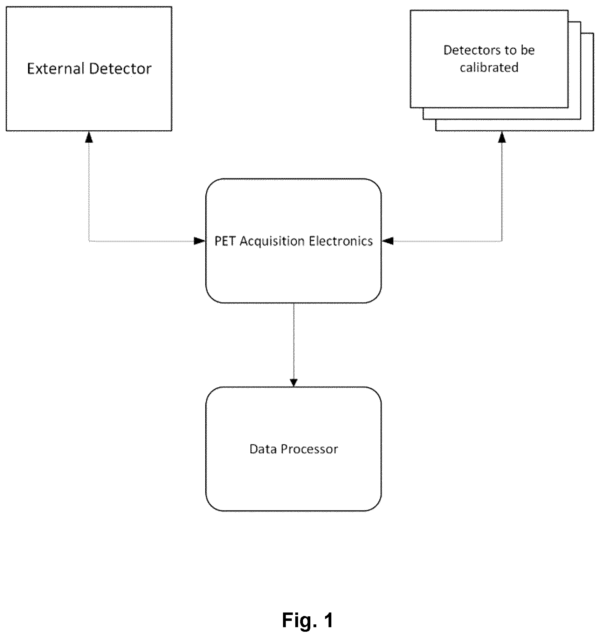

**FIG. 1** shows a diagram with the interaction of the different components for data acquisition and processing.

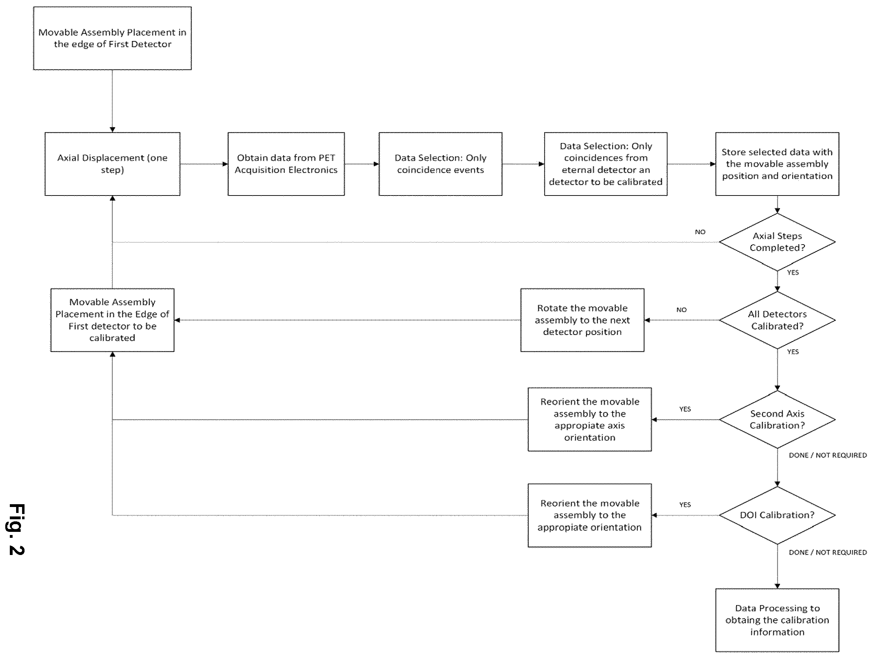

**FIG. 2** shows a flow diagram of the method of the present invention.

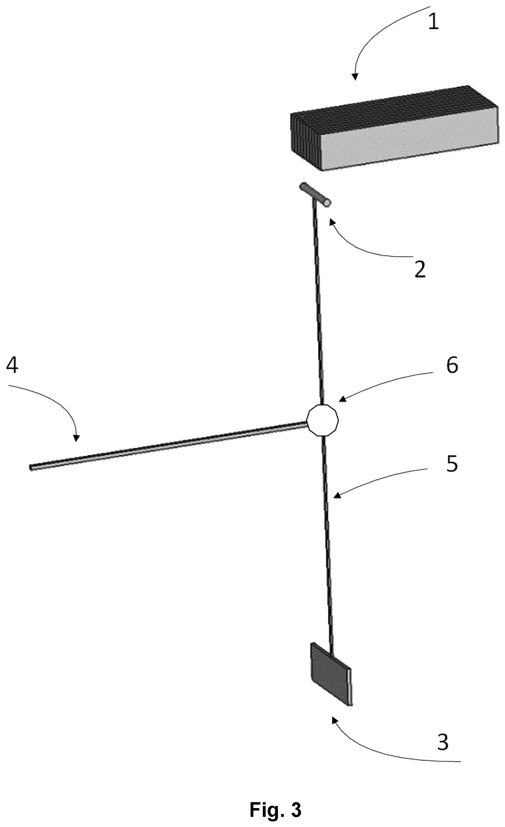

**FIG. 3** shows an exemplary calibration device according to an embodiment of the present invention. The detector to be calibrated (1) is a semi-monolithic detector. The radioactive source 2 is located close to it and having a small thickness in the calibration axis (the monolithic side of the detector to be calibrated). The external gamma ray detector 3 is connected with the radioactive source (2) through 5. The rigid mechanical support 4 will connect the movable assembly with the mechanical means for its displacement and rotation. The rotation point 6 (a second mechanical arrangement) provides the capability for the movable assembly to modify its orientation with respect of the detector 1 to be calibrated.

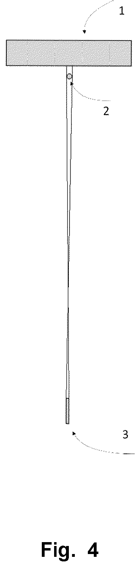

**FIG 4****.** Shows a scheme of the electronic collimation happening when the emissions from the radioactive source 2 are collimated with the external gamma ray detector 3 and impact in a specific region in the detector to be calibrated 1.
**FIG. 5** shows an exemplary calibration device according to an embodiment of the present invention including mechanical means (the articulated arm (7)) to displace axially (direction marked by the straight solid arrow) and rotate axially (orientation marked by the rotational solid arrow) of the movable assembly formed by the same components as previously described.
**FIG 6****.** shows the same calibration device with a different orientation of the mechanical movable assembly inside a possible embodiment of the plurality of detectors to be calibrated, showing the capability of the device for acquiring data in a secondary monolithic axis for its calibration.
**FIG 7****.** Shows the same calibration device with another orientation of the movable assembly, in this case non-perpendicular to the detector to be calibrated (1) showing the capability of the device to acquire data in different depths for its calibration.

### References in the figures:

1. Detector to calibrate
2. radioactive source (very thin along the axis to calibrate)
3. External gamma ray detector of the calibration device that has to be connected to the acquisition electronics (at least the clock signal) of the complete system to be calibrated
4. Mechanical rigid connection (rigid mechanical support) of the mechanical movable assembly with the first mechanical arrangement, allowing the movement of the complete set as a unit along the space directions marked with the solid arrows (translation axes Z and X) and rotation around Z axis
5. Mechanical rigid connection for the radioactive source 2 and for the calibration device detector 3 to form the mechanical movable assembly. Elements 2 and 3 have to be perfectly aligned and be associated with each other.
6. second mechanical arrangement to align the radioactive source (2) and external gamma ray detector (3) as a unit with the different calibration axis of the detector to be calibrated (1).

The mechanical movable assembly comprises the elements numbered as 2, 3, 4, 5 and 6.

### EXAMPLE

An example of a calibration device according to the invention is a device comprising a robotic arm that has can move around 6 axes, that holds the movable assembly comprising the radioactive source and the external gamma ray detector, as shown in Figure 3.

The radioactive source is a capillary fillable source of F18, with an internal diameter of 0.3mm in the calibration direction and a length in the perpendicular direction of calibration enough to cover the perpendicular area of the detector to be calibrated

The external gamma ray detector is a thin scintillator (1mm in the calibration direction), with a length on the perpendicular direction of calibration enough to cover the perpendicular area of the detector to be calibrated.

The plurality of detectors to be calibrated (scanner) consists of 8 detectors in a ring shape based on monolithic scintillator crystals (thus with two monolithic axes) and SiPM as photosensors and a PET acquisition electronics, having one monolithic side of the detectors oriented on the axial axis of the scanner.

The external gamma ray detector being connected to the acquisition electronics as another detector.

The robot has the capability to move the mechanical movable assembly composed by a thin radioactive source (2) and the external gamma ray detector (3) that are connected with rigid parts (5) in the axial direction to cover the full area of the detector to be calibrated (1). Also it can provide rotation along the axial axis to calibrate other detectors that could be in the complete system to be calibrated.

The calibration procedure is described as:
1. The radioactive source is located as close as possible to the detector to be calibrated.
2. The external gamma ray detector is located as far as possible from the radioactive source.
   Obtaining data for an axis of one detector:
   3. Locating the radioactive source and external gamma ray detector assembly at the edge of the first detector to be calibrated, with the thinner dimensions of both perpendicular to the axial direction (as figure 3), using the robot.
   4. obtaining event data for a plurality of scintillation events
   5. selecting only the proper events for calibration by means of electronic collimation,
   6. detecting and storing coincidences between the detector to be calibrated and the external gamma ray detector for this specific position and detector.

Obtaining data for the first monolithic direction:
7. Move the mechanical movable assembly on the axial axis in discreet steps of 0.5mm, repeating 4-6 for each step, using the robot.
8. Repeat step 3 and rotate the mechanical movable assembly on the axial axis until it is aligned with the border of another detector to be calibrated (steps of 45 degrees as there are 8 detectors). Repeat steps 4-7 to obtain data from all detectors, using the robot.

Obtaining data for the second monolithic direction to be calibrated:
9. Repeat step 3 and rotate the orientation of the radioactive source and external gamma ray detector with respect to the detector to be calibrated to align with the second monolithic axis to be calibrated (as figure 6, using (6)).
10. Repeat steps 4-7 to obtain data from the complete axis for all detectors to be calibrated.

Obtaining data for depth-of-interaction for all detectors:
11. Repeat step 3 and rotate the orientation of the radioactive source and external gamma ray detector with respect to the detector to be calibrated to obtain an angle of 45° between the surface of the detector to be calibrated and the assembly (as figure 7, using (6)).
12. Repeat steps 4-8 to obtain data from the complete axis for all detectors to be calibrated.

### Data Processing:

13. Process the data obtained for each detector
a. Use the data obtained with the radioactive source and external detectors aligned with one of the monolithic axes of the detectors to be calibrated, linking event data for a scintillation event to a spatial region where this scintillation event took place using a multilayer perceptron (neural network)
b. Use the data obtained with the movable assembly aligned the other of the monolithic axis of the detectors to be calibrated, linking event data for a scintillation event to a spatial region where this scintillation event took place using a multilayer perceptron (neural network)
c. Use the data obtained with the movable assembly rotated 45° from the axial axis, linking event data for a scintillation event to a plurality of DOI regions using a multilayer perceptron (neural network).

The neural networks generated will be able to calibrate any data acquired to a position in the monolithic axes (x-y) and a depth-of-interaction (z).

## Claims

1. A calibration method for a PET apparatus comprising a set of paired opposing PET detectors, the method comprising:
1) - disposing a mechanical movable assembly comprising a radioactive source that emits positrons and an external gamma ray detector, where the radioactive source is located close to, and aligned with, a first detector of the PET apparatus to be calibrated,
2) - remote positioning of the gamma ray detector close to a second detector of the PET apparatus which is opposite to the first detector, the gamma ray detector being on an axis with the radioactive source that is perpendicular to an axis of calibration,
3) - exposing the detector to be calibrated and the external gamma-ray detector to radiation of the radioactive source,
4) - shifting the mechanical movable assembly by means of a first mechanical arrangement, allowing the movement of the radioactive source and the external gamma ray detector as a unit, along the axis of calibration, in a number of discrete steps, for each step:
5) - obtaining coincidence event data of the radioactive source, from the detector to be calibrated and the external gamma ray detector,
6) - recognizing and storing coincidence t events between the detector to be calibrated and the external gamma ray detector,
7) - linking event data to a spatial region in the scintillation crystal of the detector to be calibrated, and
8) - moving the mechanical movable assembly in the PET apparatus as a unit such that the radioactive source comes close to another detector to be calibrated, repeating steps 4-7 for each detector to be calibrated in the PET apparatus.

2. The method according to claim 1, further comprising step 9) in order to calibrate another detector axis, step 9) comprises rotating the mechanical movable assembly as a unit, aligning them with a second calibration direction, and repeating steps 4-8 to calibrate the complete set of detectors in their final geometry for their use.

3. The method according to claim 1, or 2 further comprising step 10) in order to calibrate the depth-of-interaction, step 10) comprises rotating the mechanical movable assembly as a unit, aligning them in a direction not-perpendicular to surface of the detector to be calibrated, repeating steps 4-7 to calibrate all the detectors in the final geometry for their use.

4. The method according to claim 1, wherein one or more additional calibration devices are used, such that each one has a mechanical assembly comprising a radioactive source and an external gamma ray detector in the appropriate orientation to calibrate a specific axis and the corresponding mechanical assembly only moves axially and rotate transaxially.

5. A calibration device to carry out the method defined in any of claims 1 to 4, for a group of gamma detectors of a PET apparatus arranged in the final geometry for their use, the device comprising:
- a radioactive source, with a thickness in the axis of the calibration smaller than the required calibration precision, an external gamma ray detector which forms a movable assembly with the radioactive source,
- a first mechanical arrangement with capacity to move the movable assembly, allowing the axial displacement and axial rotations of the assembly,
- a coincidence processor to detect and store coincidence events between the detector to be calibrated and the external gamma ray detector.
- a data processor to take into account the acquired data at each calibration step and generates position calibration information for the calibrated detector, and
- a rigid mechanical support that connects the mechanical movable assembly with the mechanical means for its displacement and rotation
- a second mechanical arrangement with the capacity to change the orientation of the movable assembly with respect to a first detector to be calibrated.

6. The calibration device according to claim 5, wherein the radioactive source is selected from a point, a line source and a liquid source concentrated, that emits positron or gamma rays and it
has a thickness in the axis of the calibration smaller than the required calibration precision.

7. The calibration device according to any of claims 5 or 6, further comprising a data processor capable of using acquired data at each calibration step and generating position calibration information for the calibrated detector.

8. The calibration device according to claim 7, wherein the data processor is based on one of the following techniques:
▪ Center of gravity approach,
▪ Look-up tables.
▪ Neural Networks or AI approaches,
▪ Statistical algorithms.
▪ Function fits.

9. The calibration device according to any of claims 5 to 8, which further comprises an acquisition system capable of acquiring all the singles information for both the electronic readout and the PET apparatus with a common reference clock.

10. The calibration device according to any of claims 5 to 9, wherein the first mechanical arrangement is an articulated robotic arm.

11. The calibration device according to any of claims 7 to 10, wherein the data processor is capable of using the acquired data to calculate the PET coincidence events and determine the time skew of a detector after a scan of the detector surface.

12. The calibration device according to any of claims 7 to 11, wherein the data processor is capable of using the acquired data to calculate the PET coincidence events and determine the energy resolution of a detector after a scan of the detector surface.

13. The calibration device according to any of claims 7 to 12, wherein the data processor is capable of using the acquired data to calculate the PET coincidence events and determine the neural network parameters of a neural network model that provides the planar position of the impacts of a detector after a scan of the detector surface.

14. The calibration device according to any of claims 7 to 13, wherein the data processor data is capable of using the acquired data to calculate the PET coincidence events and determine the neural network parameters of a neural network model that provides the DOI of the impacts of a detector after a scan of the detector surface, having the external gamma ray detector rotated in one of the transaxial axes.

15. The calibration device according to any of claims 5 to 14, wherein the calibration device is capable of calculating alternative positioning models, preferably selected from center-of-gravity, machine learning algorithms, look-up tables or statistical algorithms.
